# EUROPÄISCHE PATENTSCHRIFT

(11) **EP 0 879 017 B1**
(45) Veröffentlichungstag und Bekanntmachung des Hinweises auf die Patenterteilung: **17.03.2004**
(21) Anmeldenummer: 97902323.1
(22) Anmeldetag: 04.02.1997
(51) Int. Cl.: A61B 5/15

(54) **SCHNEIDEVORRICHTUNG FÜR HAUT ZUR SCHMERZARMEN GEWINNUNG KLEINER BLUTMENGEN**
SKIN CUTTER FOR PAINLESS EXTRACTION OF SMALL BLOOD AMOUNTS
DISPOSITIF POUR L'INCISION DE LA PEAU PERMETTANT LE PRELEVEMENT INDOLORE DE SANG EN FAIBLES QUANTITES

(30) Priorität: 06.02.1996 DE 19604156
(43) Veröffentlichungstag der Anmeldung: 25.11.1998
(73) Patentinhaber: Roche Diagnostics GmbH, 68298 Mannheim (DE)
(72) Erfinder: BÖCKER, Dirk, D-69115 Heidelberg (DE); FRUHSTORFER, Heinrich, D-35037 Marburg (DE)
(86) Internationale Anmeldenummer: PCT/EP1997/000501
(87) Internationale Veröffentlichungsnummer: WO 1997/028741

(56) Entgegenhaltungen:
- WO-A-95/01754
- US-A- 4 203 446
- US-A- 4 924 879
- US-A- 5 304 193
- US-A- 5 318 584

## Beschreibung

Die vorliegende Erfindung betrifft eine Schneidvorrichtung für Haut zur schmerzarmen Gewinnung kleiner Blutmengen aus menschlichem oder tierischem Gewebe, beinhaltend eine Klinge mit einer Schnittkante, die eine Länge von weniger als 10 mm besitzt und einen Oszillator, der die Klinge im wesentlichen parallel zur Schnittkante in eine Schwingbewegung versetzt.

Um für Diagnosezwecke eine geringe Menge Blut aus dem Finger oder dem Ohrläppchen zu entnehmen, werden in der arztlichen Praxis Lanzetten verwendet, die manuell oder mit einer einfachen Apparatur von dem Arzt oder Laborpersonal in das entsprechende Körperteil gestochen werden. Die Lanzette muß selbstverständlich scharf und steril sein. Im übrigen sind aber in der arztlichen Praxis keine besonders hohen Anforderungen zu stellen, weil die Blutgewinnung bei einzelnen Patienten in großen zeitlichen Abständen durchgeführt werden und der Einstich durch trainiertes, speziell ausgebildetes Personal ausgeführt wird.

Wesentlich hoher sind dagegen die Anforderungen bei Blutlanzettenvorrichtungen, die zur Handhabung durch den Patienten selbst bestimmt sind. Sie werden vor allem benötigt, um im Rahmen des sogenannten "home monitoring" besonders gefährdeten Patientengruppen eine regelmäßige Uberwachung bestimmter analytischer Werte ihre Blutes zu ermöglichen.

Dies gilt insbesondere für Diabetiker, die ihren Blutzuckerspiegel häufig und regelmäßig kontrollieren sollten, um durch Anpassung der Insulininjektionen an den Bedarf, der von der Nahrungsaufnahme, der körperlichen Aktivität und anderen Faktoren abhängt, möglichst ständig innerhalb bestimmter Sollgrenzen zu halten. Dies ist für die Gesundheit solcher Patienten und die Vermeidung schwerwiegender Spätschäden, wie beispielsweise Erblindung und Amputation von Körperteilen, von allergrößter Bedeutung.

Aus diesem Grunde wurden kleine, einfach zu bedienende und verhältnismäßig kostengünstige Analysesysteme entwickelt, die meist aus Blutteststreifen und einem zugehörigen Auswertegerät bestehen. Obwohl heute die Möglichkeit zur Analyse jedem Patienten problemlos und relativ kostengünstig zur Verfügung gestellt werden kann, hat die Selbstkontrolle der Blutzuckerwerte noch nicht die wünschenswerte allgemeine Verbreitung unter Diabetikern gefunden. Ein Hauptgrund dafür sind die Schmerzen, die mit der Erzeugung der für die Blutgewinnung erforderlichen Stichwunde verbunden sind.

Im Stand der Technik sind Lanzettenvorrichtungen bekannt, bei denen eine Lanzette durch eine Feder mit hoher Geschwindigkeit in die Haut einsticht. Aus der hierdurch erzeugten Wunde tritt Blut aus, das zur Durchführung eines diagnostischen Tests verwendet werden kann. Vorrichtungen dieses Typs sind beispielsweise aus den US-Patenten US-4,203,446 und US-4,895,147 bekannt. Eine Vielzahl solcher Vorrichtungen mit unterschiedlich geschliffenen Lanzetten und unterschiedlichen Federmechanismen sind im Markt erhältlich. Es hat sich jedoch herausgestellt, daß der Schmerz, der durch diese Art von Vorrichtung erzeugt wird, nicht unter eine Grenze abgesenkt werden kann, die für den Benutzer noch deutlich als unangenehm empfunden wird. Bei Diabetikern kommt erschwerend hinzu, daß die Tests sehr häufig durchgeführt werden müssen und auf den zugänglichen Hautpartien, insbesondere den Fingerbeeren, Verhornungen entstehen, die größere Stichtiefen erforderlich machen und somit den durch die Stechvorrichtungen verursachten Schmerz steigern.

Es wurden zahlreiche verschiedene Blutlanzettenvorrichtungen entwickelt, die geeignet sein sollen, die für die Blutgewinnung erforderliche Stichwunde auf einfache Weise und verhältnismäßig schmerzarm zu erzeugen. Beispiele sind in den Patentschriften US-4,442,836, US-4,469,110, US-4,535,769 und US-4,924,897 beschrieben. Blutgewinnungsgerät und Lanzette sind meist aufeinander abgestimmt und werden auch als Blutgewinnungssystem bezeichnet. Trotz einiger Fortschritte ist auch bei diesen für die Benutzung durch den Patienten selbst bestimmten Blutlanzettenvorrichtungen der durch den Einstich erzeugte Schmerz noch zu groß.

Durch Blutlanzettenvorrichtungen, bei denen die Nadel kontrolliert geführt wird (US-4,924,897 und US-5,318,584), konnte die Schmerzhaftigkeit des Einstiches verringert werden. Solche Vorrichtungen werden als zwangsweggesteuert bezeichnet und stehen im Gegensatz zu sogenannten ballistischen Systemen, bei denen die Lanzette in relativ unkontrollierter Weise über einen Federmechanismus auf die Hautoberfläche geschleudert wird.

Aufgabe der vorliegenden Erfindung war es, eine Vorrichtung vorzuschlagen, die einen geringeren Schmerz bei der Blutgewinnung hervorruft, als dies bei Lanzettenvorrichtungen des Standes der Technik der Fall ist. Weiterhin war es Aufgabe der Erfindung ein Blutlanzette zur Verfügung zu stellen, mit der die Eindringtiefe in das Gewebe genauer und besser reproduzierbar möglich ist als mit bekannten Vorrichtungen.

Die erfindungsgemäße Lösung der vorgenannten Aufgabe besteht darin, daß zum einen ein Penetrationsvorgang der Haut durchgeführt wird, der im wesentlichen ohne eine Druckwelle senkrecht zur Haut abläuft und bei dem zum anderen durch die präzise Führung und verhältnismäßig geringe Einstichtiefe der Lanzette/Klinge relativ wenige Schmerzrezeptoren erregt werden. Dies läßt sich dadurch erreichen, daß die Haut mit einem Schneidvorgang, der im wesentlichen parallel zur Hautoberfläche erfolgt, penetriert/eröffnet wird. Dieser Schneidvorgang ist deutlich schmerzarmer als ein Stichvorgang.

Erfindungsgemäß wird eine Schmerzreduktion bei der Gewinnung kleiner Blutmengen dadurch erreicht, daß der Bewegung (in der Regel einer Linearbewegung) der Lanzette bzw. Klinge, die zum Eindringen in das Gewebe notwendig ist, eine schnelle Schwingbewegung uberlagert wird. Die Auslenkung der Klinge, die durch diese Schwingbewegung hervorgerufen wird, kann prinzipiell quer zum Gewebe oder auch im wesentlichen senkrecht zum Gewebe erfolgen. Für diese beiden Ausführungsarten haben sich jedoch unterschiedliche Klingenformen als günstig erwiesen, so daß die Ausführungsformen im Folgenden getrennt voneinander beschrieben werden.

Vorteilhaft ist bei der Kopplung einer langsamen Einstichbewegung mit einer Oszillation, daß trotz einer extrem hohen Relativbewegung der Klinge zur Haut eine tiefer in das Gewebe hineinreichende Druckwelle fast gänzlich vermieden wird, da die Auslenkung der Klinge trotz der hohen Geschwindigkeit sehr klein gehalten werden kann (vorzugsweise etwa im Bereich von 10 - 200 µm) und quasi keine Druckkomponente senkrecht zur Gewebeoberfläche erzeugt wird. Die Klinge "fällt" sozusagen in die Haut. Das Penetrationsprinzip beruht also auf einem Schneidvorgang, der durch die "vibrierende" Klinge ohne wesentliche Druckkomponente senkrecht zur Bewegungsrichtung ausgeführt werden kann. Der Schnitt kann durch den nur sehr kleinen Hub der Klinge auf kleine Dimensionen begrenzt und somit gut kontrolliert werden.

Die Kopplung einer langsamen Klingenbewegung mit einer Oszillation führt zu Schneidvorgängen, die schmerzärmer sind als die im Stand der Technik bekannten Verfahren zur Gewinnung kleiner Blutmengen.

Es wurde nach langwierigen Untersuchungen herausgefunden, daß die Nachteile der im Stand der Technik bekannten Blutlanzetten wahrscheinlich durch die Schmerzhaftigkeit des Hautpenetrationsvorganges verursacht werden, die im wesentlichen auf die Erregung von Schmerzrezeptoren und deren afferente Signale zurückgehen. Beim Auftreffen der Lanzette auf das Gewebe wird eine Druckwelle vornehmlich in Bewegungsrichtung erzeugt. Diese Druckwelle läuft der eintretenden Klinge voraus und erregt so auch zusätzlich Schmerzrezeptoren, die durch den reinen Schneid- bzw. Reißvorgang der Lanzette beim Penetrationsvorgang bzw. Schneidvorgang nicht erregt werden. Durch Vermeidung einer Druckwelle beim Auftreffen der Lanzette auf die Haut kann die Schmerzempfindung somit signifikant reduziert werden. Daneben sind wahrscheinlich die nicht definierte Eindringtiefe und unkontrollierte Bewegungsabläufe der Lanzette weitere Gründe für einen unnötig hohen Schmerz bei Nutzung der bekannten Systeme.

Eine weitere Verbesserung erfinderungsgemäßer Vorrichtungen kann erreicht werden, wenn die erzeugte Hautöffnung möglichst klein ist, vorzugsweise kleiner als 1,5 mm, noch besser kleiner als 1 mm und die Schnittbewegung sehr kontrolliert durchgeführt wird.

Es ist auch von Bedeutung, daß die eröffnete Stelle durch einen Schnitt mit einer kontrollierten Bewegung und nicht durch einen unkontrollierten "Riß" erzeugt wird. Ein "Reißen" des Gewebes würde eine entsprechende Druckwelle bzw. ein Ziehen des Gewebes zur Folge haben und damit Schmerzen hervorrufen. Als vorteilhaft haben sich Bewegungsabläufe erwiesen, bei denen die Klinge so geführt wird, daß ihre Bahn bei Hin- und Rückbewegung gleich ist. Günstig ist es auch, den Bewegungsablauf so zu steuern, daß die Hinbewegung nach Erreichen eines vordefinierten Totpunktes in eine Rückbewegung übergeht.

Eine genauere Beschreibung der Erfindung erfolgt mit Hilfe der folgenden Figuren:
- Figur 1:: Prinzipieller Zusammenhang zwischen Eindringtiefe und Schnittbreite bei Verwendung spitzer Lanzetten
- Figur 2:: Anordnung von Klinge und Gewebepartie zueinander
- Figur 3: Manuell bedienbare Blutlanzette des Typs Vampir
- Figur 4:: Blutlanzette mit Feder als Vorschubmechanismus
- Figur 5:: Blutlanzette mit Exzenter als Vorschubmechanismus
- Figur 6:: Klingentypen
- Figur 7:: Querschnitte von Klingentypen
- Figur 8:: Prinzipdarstellung einer Blutlanzette des Typs Mücke
- Figur 9:: Manuell bedienbare Blutlanzette des Typs Mücke
- Figur 10:: Koordinatensystem zur Definition der Bewegungsrichtungen
- Figur 11:: Darstellung des Zusammenhanges von Austrittsweite (A), Deformation (D) und Eindringtiefe (E)

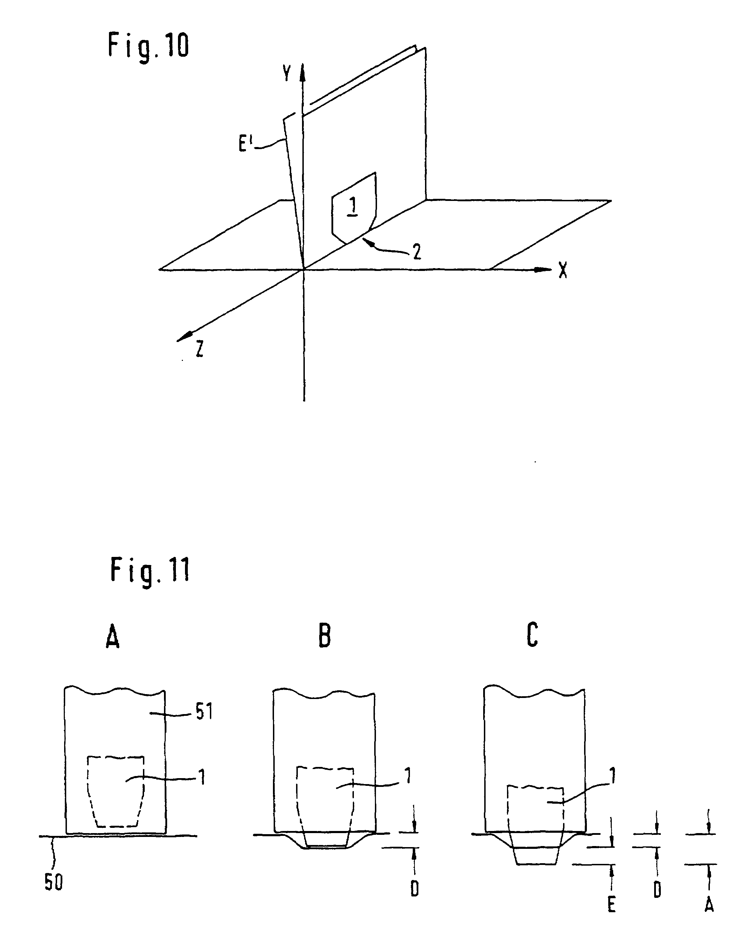

Zur Definition der Bewegungsrichtungen der Lanzette ist in Figur 10 ein Koordinatensystem dargestellt. Die Oberfläche des Gewebes liegt in der XZ-Ebene. Zum Eindringen in das Gewebe muß die Lanzette eine Bewegung ausführen, die einen Y-Anteil besitzt, d. h. die Bewegung muß einen Anteil senkrecht zur Gewebeoberfläche besitzen. Im dargestellten Fall befindet sich die Schnittkante (2) auf der Z-Achse und die Klinge (1) innerhalb der YZ-Ebene. Die Klinge kann nun senkrecht, d. h. in Richtung der negativen Y-Achse, in das Gewebe geführt werden oder sie kann auch innerhalb einer Ebene E' bewegt werden, die gegen die YZ-Ebene verkippt ist. Die Bezeichnung "im wesentlichen senkrecht zur Gewebeoberfläche" für die Bewegungsrichtung soll auch noch solche Bewegungen einschließen, die beispielsweise um 30° gegenüber der YZ-Ebene verkippt sind. Bei einer ersten Ausführungsform wird die Klinge in eine Oszillationsbewegung quer zur Bewegungsrichtung versetzt. Im Koordinatensystem der Figur 10 ist dies eine Oszillation der Klinge in Z-Richtung.

Es wird weiterhin ein Verfahren zur Gewinnung kleiner Blutmengen beschrieben, bei dem die Klinge in eine Schwingbewegung versetzt, an das zu schneidende Gewebeteil herangeführt, im wesentlichen senkrecht in das Gewebe hineingeführt und aus dem Gewebe herausgeführt wird. Dies kann einerseits durch eine aktive Bewegung der Klinge in Richtung auf das Gewebe oder auch durch eine aktive Bewegung des Gewebeteiles erfolgen. Weiterhin muß sich die Klinge beim Hautkontakt nicht bereits in Oszillation befinden, sondern die Oszillation kann auch erst nach Kontaktierung von Klinge und Gewebe beginnen.

In fast allen Bereichen des Körpers besitzt der Mensch eine sogenannte Plattenhaut, aus der eine Blutgewinnung mit Lanzetten nicht oder nur schwer möglich ist, da sich diese Haut wegen ihrer hohen Retraktionsfähigkeit nach dem Einstich oder Einschnitt in sehr kurzer Zeit verschließt, so daß das aus den verletzten Gefäßen austretende Blut nicht die Hautoberfläche erreichen kann und in subcutane Gewebeareale abgegeben wird, was zu sogenannten (Micro)-Hämatomen führt. Zur Gewinnung von Blut geeignete Hautpartien befinden sich an den Fingerkuppen, Zehenkuppen sowie den Ohrläppchen. An den Finger- und Zehenkuppen besitzt der Mensch eine sogenannte Leistenhaut. Die oberste Schicht dieser Haut ist die Epidermis, die eine Dicke von 0,1 bis 0,5 mm aufweist. Unterhalb der Epidermis beginnt das Corium, bestehend aus dem Stratum papillare und dem Stratum reticulare. Unterhalb des Coriums liegt die Subcutis. Für eine schmerzarme Gewinnung kleiner Blutmengen ist insbesondere der obere Teil des Coriums, das Stratum papillare, von Bedeutung. Das Stratum papillare ist durchzogen von einem feinen Netz von eng benachbarten Blutgefäßen und Schmerzrezeptoren. Die Blutgefäße können in diesem Gewebebereich in Kapillaren und Mikrogefäße unterteilt werden. Die Kapillaren besitzen die Form einer Haarnadel, die sich in den Hautpapillen widerspiegelt. Das arterielle Blut steigt aus dem Innern des Gewebes zur Krümmung der Haarnadel, empor, gibt dort den Sauerstoff an das umliegende Gewebe ab und wird als venoses Blut wieder in tiefer liegende Gewebeschichten über das venöse Gefäßsystem zurückgeführt. Im Rahmen der Erfindung wurde festgestellt, daß es aufgrund der anatomischen Gegebenheiten ausreicht, die Epidermis zu durchdringen und entweder einige der haamadelförmigen Blutgefäße des Stratum papillare zu durchtrennen oder/und einige der in dieser Gewebsregion vorkommenden Mikrogefäße zu zerschneiden, um für die angestrebten Diagnosezwecke eine ausreichende Blutmenge (ca. 5 µl bis 30 µl) zu erhalten.

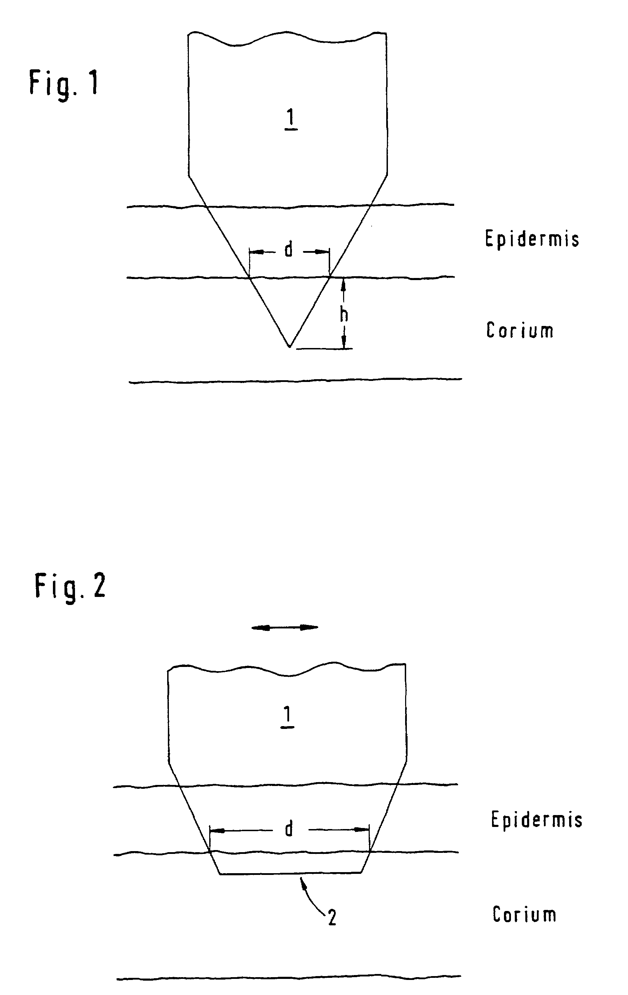

Im Stand der Technik bekannte Lanzettenvorrichtungen dringen jedoch wesentlich tiefer und zudem unkontrollierter, d. h. mit starken Bewegungen senkrecht zur Vorschubsrichtung in das Corium ein. Der Grund hierfür ist der Figur 1 zu entnehmen. Um an der Oberfläche des Coriums eine Wunde der Breite d zu erzeugen, muß die Spitze der Lanzette um die Tiefe h in das Corium eindringen. Eine Verringerung dieser Tiefe h ist mit der im Stand der Technik bekannten Technologie nur möglich, wenn die Spitze der Lanzette flacher gewählt wird, daher das Verhältnis h/d verringert wird. Stumpfere Lanzetten rufen jedoch, wie experimentell gezeigt werden konnte, einen stärkeren Einstichschmerz hervor

### ** Erste Ausführungsform (Vampir)

Erfindungsgemäß wird der Einstichschmerz dadurch erniedrigt, daß die Klinge in eine schnelle Schwingungsbewegung versetzt wird und die schwingende Klinge relativ langsam, vorzugsweise langsamer als mit 2 km/h, in das Gewebe gesenkt wird. Bei der ersten Ausführungsform der Erfindung wird eine Oszillationsbewegung der Klinge verwendet, die im wesentlichen parallel zur Schnittkante ist.

Figur 2 zeigt prinzipiell die Anordnung von Klinge und Gewebepartie zueinander. Die dargestellte Klinge (1) besitzt eine Schnittkante (2), die eine Schwingbewegung in Richtung des dargestellten Doppelpfeiles durchführt. Wird diese Klinge, während sie oszilliert, an die Epidermis herangeführt, so vermag sie die Epidermis zu durchdringen, ohne daß dabei ein nennenswerter Schmerz auftritt. Aufgrund der Oszillationsbewegung wird die Epidermis aufgeschnitten und die Geschwindigkeit, mit der die Klinge in Epidermis und Corium abgesenkt wird, kann im Gegensatz zu heute üblichen Stechlanzetten sehr klein gewählt werden. Aus Figur 2 ist zu erkennen, daß im Rahmen dieser Ausführungsform eine Klingenform verwendet werden kann, mit der bei sehr kleiner Eindringtiefe in das Corium ein ausreichend breites Teilstück d des Gewebes zerschnitten wird. Aufgrund der Anordnung der haarnadelförmigen Blutgefäße und anderer in dieser Region vorkommender Microgefäße im Corium werden diese durch die Oszillationsbewegung der Klinge effektiv durchtrennt. Zur Gewinnung einer ausreichenden Blutmenge ist lediglich eine Schnittbreite d von 300 bis 900 µm erforderlich. Zur Gewinnung größerer Blutmengen können erfindungsgemäß natürlich entsprechend längere Klingen verwendet werden.

### *** Oszillator

Zur Erzielung eines schmerzarmen Eindringens der Klinge in das Gewebe hat es sich als wichtig herausgestellt, daß die Klinge im wesentlichen parallel zur Schnittkante eine Schwingung durchführt, deren Frequenz oberhalb 500 Hz liegt. Noch wesentlich verbessert werden konnte die erfindungsgemäße Schneidvorrichtung, indem die Frequenz der Schwingbewegung auf über 700 Hz angehoben wurde. Besonders effiziente Schneidvorgänge werden mit Frequenzen im Kiloherzbereich bzw. oberhalb 900 Hz erreicht. Das Schmerzempfinden während des Schneideprozesses wird auch wesentlich durch die Amplitude der Schwingbewegung mitbestimmt. Es hat sich herausgestellt, daß die Amplitude der Schwingbewegungen unterhalb 500 µm liegen sollte. Vorzugsweise liegt sie unterhalb 300 µm und besonders bevorzugt unterhalb 250 µm. Oszillatoren, mit denen die beschriebenen Anforderungen realisiert werden können, sind insbesondere Piezoelemente, Ultraschallkeramiken, elektrodynamische Wandler und mechanische Wandler. Vor allem haben sich Piezoelemente als Oszillatoren bewährt, die beim Anlegen einer elektrischen Spannung Längenänderungen ausführen. Da diese Längenänderungen in der Regel nur ein oder wenige µm betragen ist zur Erzielung großerer Schwingungsamplituden eine mechanische Übersetzung notwendig. Dies kann vorteilhaft durch einen Stab erreicht werden, in Nähe dessen einem Ende ein Piezoelement angekoppelt ist und an dessen anderem Ende eine Klinge angebracht ist. Dieser Stab sollte eine möglichst geringe Masse aufweisen, um eine Dämpfung der Schwingung gering zu halten. Der Stab besteht vorzugsweise aus einem steifen Material, um eine gute Energieubertragung vom Oszillator auf die Klinge zu gewährleisten. Stab und Piezoelement sind kraftschlüssig an einer Gegenmasse befestigt. Es können jedoch vorteilhaft auch Stapel, zusammengesetzt aus einer Vielzahl von Piezoelementen, verwendet werden, bei denen sich die Längenänderungen der Einzelelemente addieren.

Mechanische Wandler, die eine Oszillation der Klinge bewirken sind sogenannte Feder-Masse-Schwinger. Hierzu gehört beispielsweise eine Stimmgabel an deren einem Schenkel sich die Klinge oder ein Halter mit Klinge befindet. Vorzugsweise wird der Schwinger in einer Resonanzfrequenz berieben, da hier die Auslenkung der Klinge besonders groß ist.

### *** Klinge

Für Schneidvorrichtungen zur Gewinnung kleiner Blutmengen sind Klingen geeignet, bei denen die Länger der Schnittkante kleiner als 10 mm ist. Für die Gewinnung von Blutmengen im Bereich von 100 µl haben sich Schnittkanten einer Länge von 0,2 bis 2 mm, vorzugsweise 0,4 bis 1,5 mm als geeignet erwiesen. Besonders geeignet sind Schnittkanten einer Länge von 0,5 bis 1,0 mm.

Als Materialien für die Klinge haben sich insbesondere die für Lanzetten im Stand der Technik gebräuchlichen Materialien, wie z. B. Stahl erwiesen. Allgemein können Metalle, Gläser, keramische Materialien und auch Kunststoffe verwendet werden. Besonders geeignet sind Kunststoffe, die im Spritzgußverfahren verarbeitet werden können und erkaltet eine ausreichende Härte aufweisen. Bei Kunststoffen kann gegebenenfalls ein Bearbeitungsschritt zum Schärfen der Klinge entfallen, wenn die Spritzgußform geeignet gewählt wird.Es hat sich herausgestellt, daß Beschichtungen der Klingen mit Stoffen, die die Reibung zwischen Klinge und Gewebe verringern, vorteilhaft sind. Durch Verringerung der Reibung zwischen Klinge und Gewebe kann der mechanische Energieeintrag in das Gewebe und somit ein Schmerz durch Erhitzung vermieden werden.

Besonders vorteilhaft ist es, wenn Stab und Klinge eine mechanische Einheit bilden, die ausgetauscht werden kann. Im medizinischen Bereich ist es aus hygienischen Gründen wunschenswert, die Einheit als Wegwerfteil zu gestalten, um sie nach jedem Schneidvorgang auswechseln zu können. Stab und Klinge können entweder aus unterschiedlichen Materialien gefertigt sein, oder vorteilhafterweise aus dem gleichen Material bestehen.

### *** Blutlanzette manuell

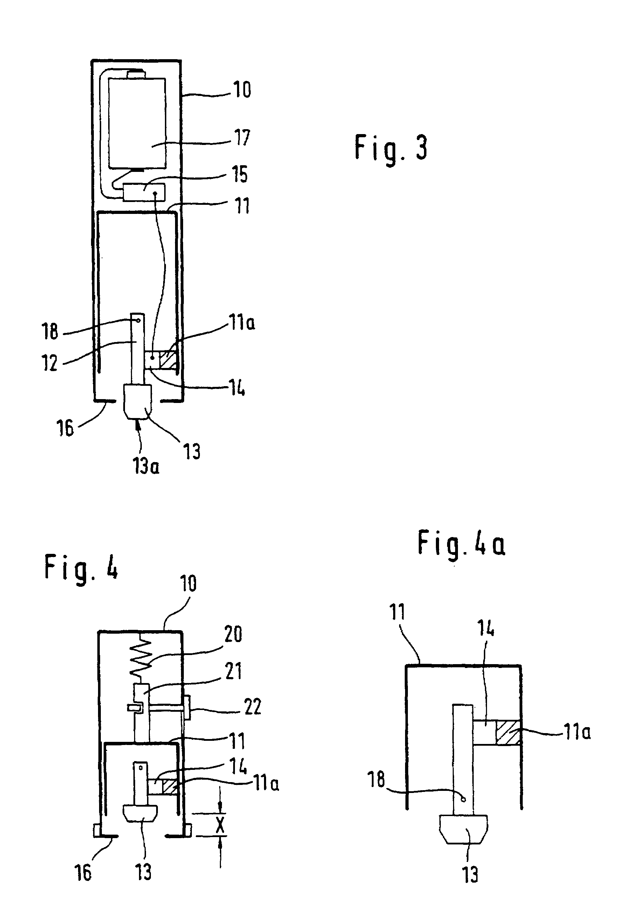

Figur 3 zeigt eine erfindungsgemaße Blutlanzette, mit einem Gehause, in dem die Klinge und der Oszillator angeordnet sind. Das Gehäuse besitzt eine äußere Hülle (10), die dem Benutzer zur Handhabung der Vorrichtung dient. Im Inneren dieser Hülle befindet sich ein Stab (12), an dem die Klinge (13) befestigt ist. Ein Piezokristall (14), der mit dem Stab (12) verbunden ist, wird über einen elektronischen Schwingungsgenerator (15) angesteuert, so daß die Klinge (13) Schwingungsbewegungen im wesentlichen parallel zur Schnittkante (13a) ausführt. Im dargestellten Beispiel befindet sich im Inneren der äußeren Hülle (10) eine weitere Hülle (11), die innerhalb der äußeren Hülle verschiebbar angeordnet ist, so daß der Bereich der Klinge, der aus der äußeren Hülle (10) herausragt, eingestellt werden kann. Der Stab (12) ist über eine Achse (18) mit der Hülle (11) verbunden, so daß dieses Ende des Stabes mit einer trägen Masse belegt ist. An ihrem unteren Ende besitzt die Hülle (10) eine Kontaktfläche (16), die manuell auf ein Gewebeteil aufgesetzt wird. Der Bereich, um den die Klinge über die Kontaktfläche hinausragt, gibt somit die Schnittiefe im Gewebe vor. Erfindungsgemäß wird die Schneidvorrichtung mit einer Einstellvorrichtung ausgerüstet, welche den maximalen und den minimalen Abstand, um den die Klinge über die Kontaktfläche hinausragt, vorgibt. Der Einstellbereich des Intervalls wird vorzugsweise so gewählt, daß seine untere Grenze größer ist als 200 µm und seine obere Grenze kleiner ist als 2500 µm. Bevorzugt ist ein Einstellbereich zwischen 0,5 und 2,0 mm oder noch besser zwischen 0,7 und 1,3 mm.

Für die Funktionsweise der Vorrichtung ist es wichtig, daß der Stab (12) aus einem Material mit ausreichend hohem Elastizitätsmodul besteht, um die Energie der Piezoschwingung auf die Klinge (13) zu übertragen. Geeignete Materialien für den Stab sind beispielsweise Glas, Federstähle, Kunststoffe und Keramiken. Weiterhin ist es wichtig, daß das Gewicht der schwingenden Bauteile (Stab und Klinge) klein ist im Vergleich zu dem Gewicht auf der gegenüberliegenden Seite des Piezoelementes. In der Figur 3 ist das Piezoelement über ein Verbindungsstück (11a) mit der inneren Hülle (11) verbunden, so daß diese als Masse wirksam ist.Besonders vorteilhaft ist es, die Apparatur so zu betreiben, daß Stab und Klinge mit einer Resonanzfrequenz schwingen.

### ** Blutlanzette mit Federtrieb

Figur 4 zeigt beispielhaft eine Ausführungsform, bei der die manuelle Bewegung, die mit der in Figur 3 dargestellten Apparatur notwendig ist, durch die Wirkung eines Federelements (20) ersetzt wird Die Feder (20) ist mit einer Schiene (21) verbunden, die eine Ausnehmung besitzt, in die ein Hebel (22) eingreift. Der Hebel (22) besitzt einen Druckknopf außerhalb des Gehäuses, so daß bei Drücken dieses Druckknopfes die Arrettierung gelöst und die innnere Hülle (11) relativ zur äußeren Hülle verschoben wird. Dadurch gelangt die Klinge (13) außerhalb des äußeren Gehäuses. Durch Variation des Abstandes X zwischen dem Rand des inneren Gehäuses (11) und der Innenseite der Kontaktfläche (16) in der Ausgangsposition kann die Schnittiefe reguliert werden, um die die Klinge in das Gewebe eindringt. Dies ist beispielsweise durch Variation der Länge der äußeren Hülle, z. B. durch eine Schraube, möglich. Weitere Möglichkeiten, die Eindringtiefe in das Gewebe zu variieren sind aus US-4,895,147 und US-5,318,584 bekannt. Die Anordnung kann beispielsweise auch so gewählt werden, daß die Klinge feststeht und sich die Hautkontaktfläche auf der Stirnseite einer Hülse befindet, die federnd gegen einen einstellbaren Anschlag montiert ist. Die Einstellmechanik für die Einstellung der Einstichtiefe ist vorzugsweise so ausgebildet, daß sie stufenweise einstellbar ist, wobei der Stufenabstand zumindest in den oben genannten Einstelibereichen höchstens etwa 0,4 mm und mindestens etwa 0,2 mm, bevorzugt 0,3 mm beträgt. Selbstverständlich kann der Einstellbereich auch über die genannten Obergrenzen hinausgehen und somit auch größere Einstichtiefen umfassen, um den Erfordernissen der relativ wenigen Personen Rechnung zu tragen, bei denen mit den genannten niedrigen Einstichtiefen (beispielsweise wegen einer besonders dicken Hornschicht) keine ausreichende Blutmenge gewonnen werden kann. Für eine genaue Einstellung der Einstichtiefe kommt es auch darauf an, daß die Klinge in ihrer Halterung derartig präzise positioniert ist, daß die Position des Klingenendes in Richtung der Einstichbewegung relativ zu dem Klingenhalter reproduzierbar ist, wenn nacheinander mehrere Klingen in der Klingenhalterung befestigt werden.

Durch die vorteilhafte Eigenschaft der erfindungsgemäßen Vorrichtung, das Gewebe nur sehr wenig zu schieben bzw. zu verdichten bevor der Penetrationsvorgang einsetzt, kann eine sehr gute Reproduzierbarkeit der Schneidtiefe erreicht werden, die deutlich besser ist (< +/-0,1 mm) als die von heute verfügbaren Systemen. Durch diese sehr gute Penetrationsfähigkeit mit der erfindungsgemäßen Vorrichtung sind auch individuelle Variabilitäten des Hautturgors und/oder der Hautdichte bzw. der Hautelastizität nunmehr nur noch von untergeordneter Bedeutung. Dies liegt daran, daß sich das der Klinge gegenüberliegende Gewebe beim ersten Kontakt mit der vorwärts drängenden Klinge nicht verdichtet, sondern der Penetrations- bzw. Schneidvorgang bereits mit Auftreffen der Klinge auf die Haut beginnt.

In der Figur 4a ist die innere Hülse (11) aus Fig. 4 mit einer etwas abgewandelten Anordnung von Oszillator, Stab und Klinge dargestellt. Der Piezokristall (14) und die Klinge (13) sind auf unterschiedlichen Seiten der Achse (18) angeordnet. Auf diese Weise wird eine Oszillation der Klinge in der Art eines Wiegemesser erzielt. Hierbei besitzt die Schwingungsbewegung sowohl eine Komponente parallel zur Schnittkante, als auch eine Komponente senkrecht dazu. Bei Oszillation des Stabels um die Achse bewegt sich die Klinge auf dem Abschnitt eines Kreises.

In Figur 11 ist dargestellt, wie die Autrittsweite der Lanzette aus der Schneidvorrichtung und die Eindringtiefe in das Gewebe zusammenhängen. Figur 11 A zeigt den Ausgangszustand des Systems, bei dem sich die Klinge (1) vollständig im Gehäuse (51) befindet und das Gehäuse auf eine Hautpartie (50) gedrückt wird. Figur 11B zeigt einen Zustand, bei dem die Klinge die Haut verdrängt, ohne sie zu durchschneiden. Die Deformation der Haut ist mit (D) bezeichnet. Figur 11C stellt einen Zustand dar, bei dem die Klinge die Haut durchtrennt hat. Die Austrittsweite (A) der Klinge aus dem Gehäuse resultiert in einer Deformation (D) und einer Eindringtiefe (E) in das Gewebe. Die Deformation (D) kann individuell und auch beim gleichen Individium stark schwanken, so daß bei konstanter Austrittsweite die Eindringtiefe (E) ebenfalls stark schwankt, was unerwünscht ist. Bei Vorrichtungen gemäß der vorliegenden Erfindung ist die Deformation (D) aus bereits genannten Gründen sehr klein, so daß Schwankungen von (D) ebenfalls sehr klein sind und daher bei festgelegter Austrittsweite (A) die Eindringtiefe (E) in die Haut sehr genau vorbestimmt werden kann.

Die Einstichtiefe sollte durch den Benutzer selbst leicht und präzise einstellbar sein. Vorzugsweise umfaßt der Einstellbereich dabei ungewöhnlich niedrige Einstichtiefen zwischen 0,5 und 2,0 mm, wobei der Bereich zwischen 0,7 mm und 1,3 mm von besonderer Bedeutung ist. Im Rahmen der vorliegenden Erfindung wurde festgestellt, daß die in der Praxis für die Analyse benötigte Blutmenge, welche üblicherweise zwischen 1 und 50 µl, in der Mehrzahl der Fälle zwischen 5 und 30 µl liegt, bei der überwiegenden Mehrzahl der Menschen überraschenderweise bereits mit solchen niedrigen Einstichtiefen bei deutlich reduziertem Schmerz gewonnen werden kann. wenn man dafür sorgt, daß die Tiefe des Einstichs bei einer bestimmten, unveranderten Einstellung des Gerätes gut reproduzierbar ist. Bei unveränderter Einstellung der Schneidvorrichtung sollten aufeinanderfolgende Schneidvorgänge in der maximal erreichten Schnittiefe um weniger als 0,15 mm, bevorzugt 0,1 mm und besonders bevorzugt 0,05 mm variieren.

### *** Blutlanzette mit Exzenterantrieb

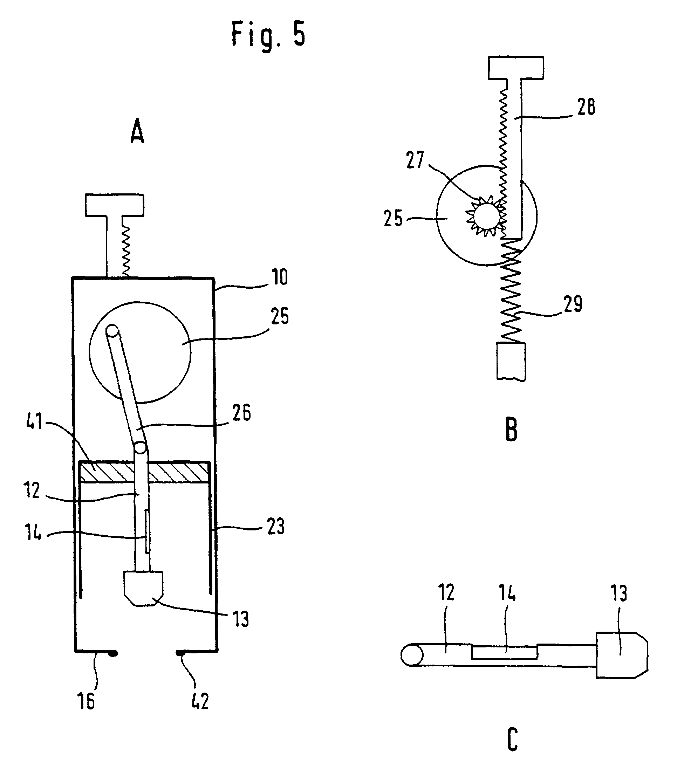

Figur 5A zeigt eine Blutlanzette, bei welcher der Stab (12), an dem sich die Klinge (13) befindet, an einem Gewicht (41) befestigt ist. Das Gewicht (41) befindet sich in einer inneren Hülse (23), die ihrerseits in der außeren Hülse (10) verschiebbar angeordnet ist. Durch das Gewicht (41) ist der Stab (12) gesteckt, der mit dem Gewicht (41) starr, beispielsweise über eine Verklebung oder Verschraubung, verbunden ist. In den Stab (12) ist ein Piezoelement (14) integriert, das den Stab (12) und die Klinge (13) in Schwingung versetzt. An der Kontaktfläche (16) der Vorrichtung, die an die Haut angedrückt wird, kann vorteilhaft ein Wuist (42) angebracht sein, der um die Öffnung zum Austritt der Klinge verläuft. Dieser Wulst dient dazu, die Hautpartie, die von der Klinge (13) geschnitten wird, zu spannen und zu fixieren. Als vorteilhaft hat es sich erwiesen, wenn der Wulst (42) um 0,2 bis 0,3 mm über die Kontaktfläche (16) hinausragt.

Figur 5A zeigt weiterhin eine vorteilhafte Antriebsmechanik für eine Bewegung der Klinge in das Gewebe. Diese Antriebsvorrichtung besitzt eine Exzenterscheibe (25), an der über eine Achse eine Hebelstange (26) befestigt ist, die ihrerseits über eine Achse mit dem Stab (12) verbunden ist. Figur 5B zeigt die Exzenterscheibe (25) von der Rückseite. Auf dieser Seite besitzt die Exzenterscheibe ein Zahnrad (27), das über eine Zahnstange (28) angetrieben wird. Die in Figur 5 dargestellte Vorrichtung besitzt den Vorteil, daß beim Drücken der Zahnstange (28) durch den Benutzer die Klinge in das Gewebe abgesenkt und auch wieder herausgezogen wird. Da der Benutzer lediglich eine einzige Druckbewegung ausführen muß, wird die Handhabung der Schneidvorrichtung erleichtert. Für die Rückbewegung der Zahnstange (28) in ihre Ausgangsposition kann ein Federelement (29) vorgesehen werden, das bei Herabdrücken der Zahnstange komprimiert wird und dazu dient, die Zahnstange in ihre Ausgangsposition zurückzubewegen. Vorteilhaft kann weiterhin ein Freilauf für das Zahnrad (27) vorgesehen werden, so daß bei der Zurückbewegung der Zahnstange in ihre Ruheposition keine Drehung der Exzenterscheibe (25) erfolgt und die Klinge nicht nochmals in das Gewebe abgesenkt wird.

Figur 5C zeigt den Stab (12) mit integriertem Piezoelement (14). Das Piezoelement ist so beschaffen, daß es beim Anlegen einer elektrischen Wechselspannung Längenänderungen entlang seiner Längsachse ausführt. Durch diese Längenänderungen findet eine Verbiegung des Stabes, analog zu einem Bimetallstreifen, statt. Mit elektrischen Signalen geeigneter Frequenz, die an das Piezoelement angelegt werden, kann eine mechanische Schwingung des Stabs (12) hervorgerufen werden.

### *** Klingentypen

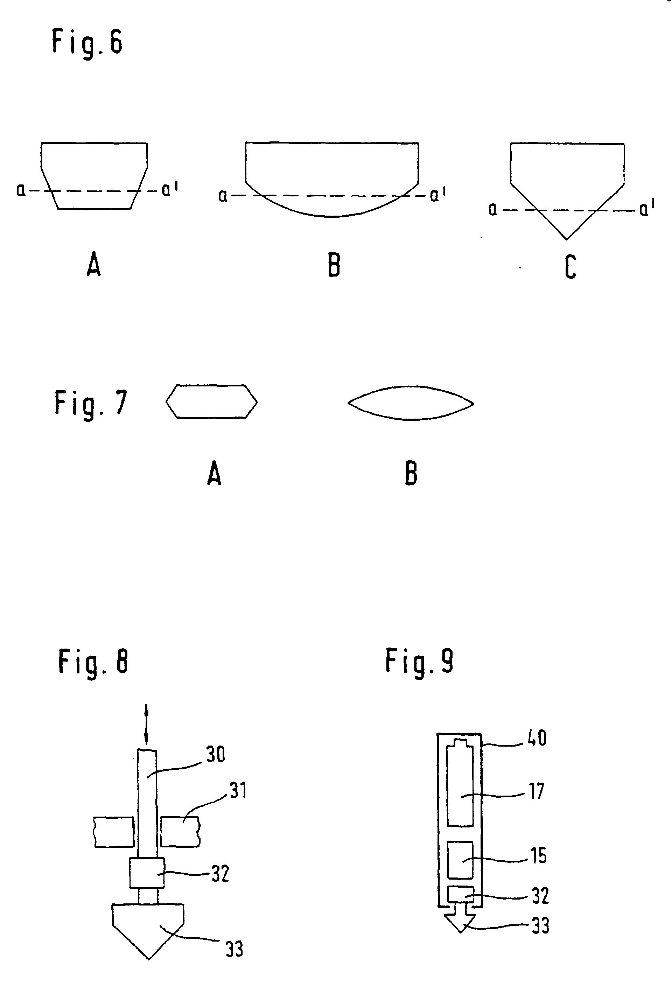

Figur 6 zeigt drei erfindungsgemäß bevorzugte Ausführungsformen von Klingen. In Figur 6A ist eine trapezförmige Klinge mit gerade Schnittkante dargestellt. Figur 6B zeigt eine wiegemesserförmige Klinge und die Klinge in Figur 6C läuft spitz zu. Jeder dieser 3 Klingentypen kann verschiedene Formen des Querschnitts besitzen. Figur 7 zeigt zwei Querschnitte A und B entlang der Schnittllinie aa', die möglich sind. Jede der in Figur 6A bis 6C dargestellten Klingen kann einen dieser beiden Querschnitte besitzen. Zur Verdeutlichung ist in den Figuren 6A bis 6C jeweils die Schnittlinie angegeben.

Die Grundformen der Klinge können beispielsweise trapezförmig, kreis- und halbkreisförmig, viereckig, vieleckig, spitz zulaufend oder wiegemesserförmig sein. Als Anschliffarten sind beispielsweise möglich:
- umlaufender Anschliff
- Facettenschliff und
- Schliff mit Gegenschliff

### ** Zweite Ausführungsform (Mücke)

Zur Erfindung gehört weiterhin eine Ausführungsform, bei der die Klinge senkrecht zur Gewebeoberfläche in eine Schwingbewegung versetzt wird. Bei dieser Ausführungsform verlaufen daher die mikroskopischen Bewegungen der Klinge durch die Schwingbewegung und die Bewegung zum Einführen der Klinge in das Gewebe im wesentlichen parallel. Bei dieser Ausführungsform können vorteilhaft spitz zulaufende Klingen und auch Klingen mit mehreren Kanten eingesetzt werden. Letzterer Typ von Klingen kann beispielsweise durch einen Facettenanschliff erreicht werden.Es können bei dieser Ausführungsform auch Nadeln verwendet werden, die spitz zulaufen und vorteilhafterweise einen Facettenschliff aufweisen.

Bei dieser Ausführungsform wird die Schwingung des Oszillators vorteilhafterweise über eine starre Verbindung an die Klinge angekoppelt, die nach Möglichkeit eine laterale Verschiebung oder Schwingung der Klinge vermeidet und nur eine vertikale Oszillation zur Hautoberfläche zuläßt. Die Schwingungsamplitude der Klinge ist vertikal zur Hautoberfläche kleiner als 300 µm, vorzugsweise kleiner als 100 µm, bevorzugt kleiner als 50 µm. Als Oszillator können auch bei dieser Ausführungsform mechanische Schwingungsgeneratoren, elektrodynamische Wandler, Piezoschwinger und dergleichen verwendet werden.

### *** Schemazeichnung

Figur 8 zeigt schematisch eine Vorrichtung, wie diese zweite Ausführungsform der Erfindung realisiert werden kann. Die Stange (30) ist verschiebbar in einer Hülse (31) angeordnet. An der Stange (30) befindet sich ein Piezokristall (32), der seine Schwingungen auf die Klinge (33) übertragt. Bei dieser Ausführungsform ist der Piezokristall so angeordnet, daß seine Schwingungen eine Bewegung der Klinge (33) in Längsrichtung der Stange gemäß dem oberhalb der Stange dargestellten Doppelpfeil hervorrufen. Dabei ist wichtig, daß das Gewicht der schwingenden Bauteile (Stab und Klinge) klein ist zur Masse auf der anderen Seite des Oszillators, d. h. auf seiten der Stange (30). Zur Durchführung eines Gewebeschnitts wird zunächst der Piezokristall (32) in Schwingungen versetzt und die Klinge (33) daraufhin durch Verschieben der Stange (30) auf das Gewebe abgesenkt. Nach Erzeugung eines ausreichend großen Schnittes wird die Stange (30) in der entgegengesetzten Richtung bewegt und die Klinge (33) aus dem Gewebe herausgezogen. Bei dieser Ausführungsform haben sich spitz zulaufende Klingen als vorteilhaft erwiesen. Insbesondere sind flache Klingen, mit einem Querschnitt gemäß den Figuren 7A oder 7B vorteilhaft. Es hat sich weiterhin herausgestellt, daß die Schwingungsfrequenz der Klinge quer zur Gewebeoberfläche oberhalb 100 Hz liegen sollte, damit die Schmerzempfindung bei dem Schneideprozeß klein ist. Durch Erhöhung der Frequenzen auf über 500 Hz ist eine weitere Verminderung des Schmerzempfindens möglich. Aus dem gleichen Grund sollte auch die Amplitude der Schwingbewegung unterhalb 100 µm, vorzugsweise unter 50 µm liegen.

Die zur Funktionsweise der Vorrichtung notwendige Schwingung der Klinge (33) wird durch eine geeignete geometrische Anordnung von Massen erreicht, mit der eine sogenannte Resonanzüberhöhung der Amplitude erzeugt wird.

Die in Figur 8 schematisch dargestellte Apparatur kann beispielsweise mit denen in den Figuren 4 und 5 beschriebenen Antriebsmechanismen zur Bewegung der Klinge quer zur Gewebeoberfläche kombiniert werden.

### ** Manuelle Stechhilfe

Figur 9 zeigt eine besonders einfache Ausführungsform, bei der sich innerhalb eines Gehäuses (40) ein elektronischer Schwingungsgenerator (15) befindet, der durch eine Batterie (17) angetrieben wird. Der Schwingungsgenerator (15) versetzt den Piezokristall (32) in Schwingungen. Die Klinge (33) ist in dieser Ausführungsform direkt an den Piezokristall angekoppelt. Der Benutzer fuhrt die schwingende Klinge (33) selbst an die zu schneidende Gewebepartie heran und führt einen Schnitt gewünschter Tiefe aus.

### Bezugszeichenliste

- (1): Klinge
- (2): Schnittkante
- (10): äußere Hülle
- (11): innere Hülle
- (11a): Verbindungsstück
- (12): Stab
- (13): Klinge
- (14): Piezokristall
- (15): elektronischer Schwingungsgenerator
- (16): Kontaktfläche
- (17): Batterie
- (18): Achse
- (19): Gewicht
- (20): Federelement
- (21): Schiene mit Ausnehmung
- (22): Hebel
- (23): Führungselement
- (24): Moosgummi

- (25): Exzenterscheibe
- (26): Hebelstange
- (27): Zahnrad
- (28): Zahnstange
- (29): Federelement
- (30): Stange
- (31 ): Hülse
- (32): Piezokristall
- (33): Klinge
- (40): Gehäuse
- (41): Gewicht
- (42): Wulst
- (50): Hautpartie
- (51): Gehäuse

## Patentansprüche

1. Blutentnahmesystem zur Gewinnung von Blut aus menschlichem oder tierischem Hautgewebe, beinhaltend
eine Klinge(1, 13) mit einer Schnittkante (2),
**dadurch gekennzeichnet, dass**
die Schnittkante eine Länge von weniger als 10 mm besitzt und
ein Oszillator die Klinge im wesentlichen parallel zur Schnittkante in eine Schwingbewegung versetzt.

2. Blutentnahmesystem gemäß Anspruch 1, die ein Gehäuse besitzt, in dem Klinge und Oszillator angeordnet sind und das eine Kontaktfläche (16) zum Inkontaktbringen mit einer Hautfläche besitzt, wobei diese Kontaktfläche eine Öffnung aufweist, die für die Klinge und deren Oszillationshub ausreichend groß ist.

3. Blutentnahmesystem gemäß Anspruch 1 und 2, bei der die Kontaktfläche zur Haut so gestaltet ist, daß um die Peripherie der Öffnung ein Wulst (42) verläuft, der die an die Kontaktfläche angedrückte Haut bzw. das Gewebe spannt und fixiert.

4. Blutentnahmesystem gemäß Anspruch 3, bei der der Wulst (42) um 0,2 bis 0,3 mm über die Kontaktfläche hinausragt.

5. Blutentnahmesystem gemäß Anspruch 1, bei der die Schwingung des Oszillators über einen Stab (12) auf die Klinge übertragen wird.

6. Blutentnahmesystem gemäß Anspruch 5, bei der im Stab ein Piezoelement (14) integriert ist, das beim Anlegen einer elektrischen Wechselspannung Längenänderungen entlang der Längsachse des Stabes ausführt.

7. Blutentnahmesystem gemäß Anspruch 5, bei dem Stab und Klinge eine auswechselbare mechanische Einheit darstellen.

8. Blutentnahmesystem gemäß Anspruch 7, bei dem Stab und Klinge einteilig aus dem gleichen Material hergestellt sind.

9. Blutentnahmesystem gemäß einem der Ansprüche 1 oder 2, bei dem die Schnittkante der Klinge eine Länge von 0,2 bis 2,0 mm, bevorzugt 0,4 bis 1,5 mm, besonders bevorzugt 0,5 bis 1,0 mm besitzt.

10. Blutentnahmesystem gemäß einem der Ansprüche 1 oder 9, bei der die Frequenz der Schwingbewegung oberhalb 500 Hz, bevorzugt oberhalb 700 Hz, besonders bevorzugt oberhalb 900 Hz liegt.

11. Blutentnahmesystem gemäß einem der Ansprüche 1, 9 oder 10, bei der die Amplitude der Schwingbewegung unterhalb 500 µm, vorzugsweise unterhalb 300 µm, besonders bevorzugt unterhalb 250 µm liegt.

12. Blutentnahmesystem gemäß Anspruch 2, bei der die Klinge (13) senkrecht zur Schnittkante verschiebbar angeordnet ist, so daß die Schnittkante (13a) in einer ersten Position im Inneren des Gehäuses angeordnet ist und in einer zweiten Position nach außen über die Kontaktfläche (16) hinausragt.

13. Blutentnahmesystem gemäß Anspruch 1, 2 oder 12, bei dem die Klinge (13) mit einem Vorschubmechanismus (25, 26) senkrecht zur Schnittkante bewegt wird.

14. Blutentnahmesystem gemäß einem der Ansprüche 1 oder 2, bei der die Klinge (13) an einem gespannten Federelement (20) befestigt ist und beim Entspannen des Federelementes quer zur Schnittkante bewegt wird.

15. Blutentnahmesystem gemäß einem der Ansprüche 1 oder 2, bei der die Klinge in Einstichrichtung feststeht und die Hautkontaktfläche an einer Hülse vorgesehen ist, die federnd gegen einen einstellbaren Anschlag montiert ist, durch den die maximale Eindringtiefe der Klinge in das Gewebe eingestellt wird.

16. Blutentnahmesystem gemäß Anspruch 13, bei der Vorschubmechanismus oder das Federelement die Klinge mit einer Geschwindigkeit von weniger als 2 km/h, bevorzugt weniger als 0,5 km/h, besonders bevorzugt weniger als 0,2 km/h senkrecht zur Schnittkante bewegt.

17. Blutentnahmesystem gemäß Anspruch 2, bei der die Länge, um die die Klinge im Laufe des Schneidvorganges maximal über die Kontaktfläche hinausragt, eingestellt werden kann und der Einstellbereich ein Intervall umfaßt, dessen untere Grenze größer ist als 200 µm und dessen obere Grenze kleiner ist als 2500 µm.

18. Blutentnahmesystem gemäß Anspruch 1, bei dem die Klinge eine der folgenden Grundformen besitzt:
- trapezförmig,
- kreis- und halbkreisförmig,
- viereckig,
- vieleckig,
- spitz zulaufend oder
- Wiegemesserform.

19. Blutentnahmesystem gemäß Anspruch 1, bei dem die Klinge eine der folgenden Anschliffarten besitzt:
- umlaufender Anschliff
- Facettenschliff
- Schliff mit Gegenschliff

20. Blutentnahmesystem gemäß Anspruch 1, bei welcher der Oszillator einen Piezokristall (14), eine Ultraschallkeramik oder einen elektrodynamischen Wandler oder ein mechanisch angeregtes Schwingelement als Schwingelement beinhaltet und dieses Schwingelement über einen elektronischen (15, 17) oder mechanischen Schwingungsgenerator angesteuert wird.

21. Blutentnahmesystem gemäß Anspruch 1, bei der die Klinge durch einen mechanischen Oszillator in Schwingungen versetzt wird.

22. Blutentnahmesystem gemäß Anspruch 1, bei dem die Klinge aus einem Kunststoff besteht, der in einem Spritzgußprozeß verarbeitbar ist.

23. Blutentnahmesystem zur Gewinnung von Blut aus menschlichem oder tierischem Gewebe, beinhaltend
eine Klinge (33) mit einer spitz zulaufenden Schnittkante
**dadurch gekennzeichnet, dass**
die Klinge im wesentlichen senkrecht zur Gewebeoberfläche durch einen Oszillator (32) in eine Schwingbewegung versetzt wird, wobei die Frequenz der Schwingbewegung zwischen 10 Hz und 10 kHz liegt.

24. Blutentnahmesystem gemäß Anspruch 23, bei dem die Klinge nadelförmig ist.

25. Blutentnahmesystem gemäß Anspruch 23, bei der die Frequenz der Schwingbewegung größer als 500 Hz, bevorzugt oberhalb 700 Hz, besonders bevorzugt oberhalb 1000 Hz, jedoch unterhalb 10 kHz liegt.

26. Blutentnahmesystem gemäß Anspruch 23, bei der die Amplitude der Schwingbewegung unterhalb 300 µm, vorzugsweise unterhalb 100 µm, besonders bevorzugt unterhalb 50 µm ist.

27. Blutentnahmesystem gemäß Anspruch 23, die eine Bewegungsvorrichtung (25, 26) zur Bewegung der Klinge im wesentlichen senkrecht zur Gewebeoberfläche besitzt, die die Bewegung der Klinge so steuert, daß die Bewegung bei Erreichen eines vordefinierten Totpunktes in die Rückbewegung übergeht.

28. Blutentnahmesystem gemäß Anspruch 23, die eine Bewegungsvorrichtung (25, 26) zur Bewegung der Klinge im wesentlichen senkrecht zur Gewebeoberfläche und eine Führung (10, 23, 41) besitzt, die die Bewegung der Klinge in das Gewebe (Hinbewegung) und die Bewegung aus dem Gewebe (Rückbewegung) so steuert, daß die Bahn der Klinge bei Hinund Rückbewegung im wesentlichen gleich ist.

29. Blutentnahmesystem gemäß Anspruch 23, bei der die Klinge an einem gespannten Federelement (20) befestigt ist und die Klinge beim Entspannen des Federelements im wesentlichen senkrecht zur Schnittkante bewegt wird.

30. Blutentnahmesystem gemäß Anspruch 27 oder 29, bei der die durch die Bewegungsvorrichtung oder das Federelement hervorgerufene Geschwindigkeit der Klinge senkrecht zur Gewebeoberfläche kleiner als 1 km/h, bevorzugt kleiner als 0,5 km/h, besonders bevorzugt kleiner als 0,2 km/h ist.

31. Blutentnahmesystem gemäß Anspruch 23, bei der die Klinge mehrere spitz zulaufende Schnittkanten besitzt.

## Claims

1. Blood collection system for obtaining blood from human or animal skin tissue comprising
a blade (1, 13) having a cutting edge (2),
**characterized in that**
the cutting edge has a length of less than 10 mm and
an oscillator vibrates the blade essentially parallel to the cutting edge.

2. Blood collection system as claimed in claim 1 which has a housing in which the blade and oscillator are located and a contact surface (16) for contact with a skin surface, said contact surface having an opening which is large enough for the blade and the stroke of the oscillation movement.

3. Blood collection system as claimed in claim 1 and 2 in which the skin contact surface is designed such that there is a bulge (42) around the periphery of the opening which tensions and fixes the skin or tissue pressed against the contact surface.

4. Blood collection system as claimed in claim 3 in which the bulge (42) projects beyond the contact surface by 0.2 to 0.3 mm.

5. Blood collection system as claimed in claim 1 in which the oscillation of the oscillator is transferred to the blade by means of a rod (12).

6. Blood collection system as claimed in claim 1 in which a piezoelement (14) is integrated in the rod which changes its length along the longitudinal axis of the rod when an alternating voltage is applied.

7. Blood collection system as claimed in claim 5 in which the rod and blade form an exchangeable mechanical unit.

8. Blood collection system as claimed in claim 7 in which the rod and blade are made as a one piece from the same material.

9. Blood collection system as claimed in one of the claims 1 or 2 in which the cutting edge of the blade has a length of 0.2 to 2.0 mm, preferably of 0.4 to 1.5 mm and particularly preferably 0.5 to 1.0 mm.

10. Blood collection system as claimed in one of the claims 1 or 9 in which the frequency of the oscillating movement is above 500 Hz, preferably above 700 Hz and particularly preferably above 900 Hz.

11. Blood collection system as claimed in one of the claims 1, 9 or 10 in which the amplitude of the oscillating movement is below 500 µm, preferably below 300 µm and particularly preferably below 250 µm.

12. Blood collection system as claimed in claim 2 in which the blade (13) is movable perpendicular to the cutting edge so that in a first position the cutting edge (13a) is located inside the housing and in a second position it protrudes outwards beyond the contact surface (16).

13. Blood collection system as claimed in claim 1, 2 or 12, in which the blade (13) is moved perpendicularly to the cutting edge by a propelling mechanism (25, 26).

14. Blood collection system as claimed in one of the claims 1 or 2 in which the blade (13) is attached to a tensioned spring element (20) and is moved transversely to the cutting edge when the tension of the spring element is released.

15. Blood collection system as claimed in one of the claims 1 or 2 in which the blade is stationary in the direction of lancing and the skin contact surface is provided on a sleeve which is spring-mounted against an adjustable stop that is used to adjust the maximum penetration depth of the blade into the tissue.

16. Blood collection system as claimed in claim 13 in which the propelling mechanism or the spring element moves the blade perpendicularly to the cutting edge at a speed of less than 2 km/g, preferably of less than 0.5 km/h and particularly preferably of less than 0.2 km/h.

17. Blood collection system as claimed in claim 2 in which the maximum length by which the blade projects beyond the contact surface during the cutting process can be adjusted and the adjustment range covers an interval whose lower limit is larger than 200 µm and whose upper limit is less than 2500 µm.

18. Blood collection system as claimed in claim 1 in which the blade has one of the following basic shapes:
- trapezoid,
- circular and semicircular,
- quadrangular,
- polygonal,
- tapered or
- shaped like a chopping blade.

19. Blood collection system as claimed in claim 1 in which the blade has one of the following types of ground surfaces:
- circumferential,
- facets
- ground finishes in opposite directions.

20. Blood collection system as claimed in claim 1 in which the oscillator comprises a piezocrystal (14), an ultrasonic ceramic or an electrodynamic transducer or a mechanically excited vibrating element as an oscillating element and said oscillating element is actuated by means of an electronic (15, 17) or mechanical oscillation generator.

21. Blood collection system as claimed in claim 1 in which the blade is vibrated by a mechanical oscillator.

22. Blood collection system as claimed in claim 1 in which the blade is made from a plastic that can be processed in an injection moulding process.

23. Blood collection system for obtaining blood from human or animal tissue comprising
a blade (33) having a tapered cutting edge
**characterized in that**
the blade is vibrated essentially perpendicular to the tissue surface by an oscillator (32) the frequency of the oscillation being between 10 Hz and 10 kHz.

24. Blood collection system as claimed in claim 23 in which the blade has a needle shape.

25. Blood collection system as claimed in claim 23 in which the frequency of the oscillation is above 500 Hz, preferably above 700 Hz and particularly preferably above 1000 Hz but below 10 kHz.

26. Blood collection system as claimed in claim 23 in which the amplitude of the oscillating movement is below 300 µm, preferably below 100 µm and particularly preferably below 50 µm.

27. Blood collection system as claimed in claim 23 which has a moving device (25, 26) for moving the blade essentially perpendicular to the tissue surface that controls the movement of the blade in such a manner that upon arriving at a predefined dead point the forward movement is converted into a return movement.

28. Blood collection system as claimed in claim 23 which has a moving device (25, 26) for moving the blade essentially perpendicular to the tissue surface and a guide (10, 23, 41) which controls the movement of the blade into the tissue (forwards movement) and its movement from the tissue (return movement) such that the path of the blade during the forwards and backwards movement is essentially identical.

29. Blood collection system as claimed in claim 23 in which the blade is attached to a tensioned spring element (20) and the blade is moved essentially perpendicular to the cutting edge when the spring element is released.

30. Blood collection system as claimed in claim 27 or 29 in which the speed of the blade perpendicular to the tissue surface due to the moving device or spring element is less than 1 km/h, preferably less than 0.5 km/h and particularly preferably less than 0.2 km/h.

31. Blood collection system as claimed in claim 23 in which the blade has several tapered cutting edges.

## Revendications

1. Système de prélèvement de sang pour obtenir du sang provenant d'un tissu cutané humain ou animal, comprenant
une lame (1, 13) avec un bord tranchant (2),
**caractérisé en ce que** le bord tranchant possède une longueur inférieure à 10 mm et **en ce qu'**un oscillateur soumet la lame essentiellement parallèlement au bord tranchant à un mouvement oscillant.

2. Système de prélèvement de sang selon la revendication 1, possédant un boîtier dans lequel sont logés la lame et l'oscillateur et possédant une surface de contact (16) pour la mise en contact avec une surface de peau, ladite surface de contact ayant une ouverture qui est suffisamment grande pour la lame et le mouvement oscillant de celle-ci.

3. Système de prélèvement de sang selon les revendications 1 et 2, dans lequel la surface de contact avec la peau est telle qu'un bourrelet (42) entoure la périphérie de l'ouverture, bourrelet qui tend et fixe la peau ou le tissu plaqué contre la surface de contact.

4. Système de prélèvement de sang selon la revendication 3, dans lequel le bourrelet (42) dépasse la surface de contact de 0,2 à 0,3 mm.

5. Système de prélèvement de sang selon la revendication 1, dans lequel l'oscillation générée par l'oscillateur est transmise à la lame par une tige (12).

6. Système de prélèvement de sang selon la revendication 5, dans lequel la tige intègre un élément piézoélectrique (14) qui en appliquant une tension électrique alternative effectue des changements de longueur suivant l'axe longitudinal de la tige.

7. Système de prélèvement de sang selon la revendication 5, dans lequel la tige et la lame constituent un mécanisme échangeable.

8. Système de prélèvement de sang selon la revendication 7, dans lequel la tige et la lame sont réalisées d'un seul tenant dans la même matière.

9. Système de prélèvement de sang selon l'une des revendications 1 ou 2, dans lequel le bord tranchant de la lame possède une longueur comprise entre 0,2 et 2,0 mm, de préférence entre 0,4 et 1,5 mm, de préférence encore entre 0,5 et 1,0 mm.

10. Système de prélèvement de sang selon l'une des revendications 1 ou 9, dans lequel la fréquence du mouvement oscillant est supérieure à 500 Hz, de préférence supérieure à 700 Hz, de préférence encore supérieure à 900 Hz.

11. Système de prélèvement de sang selon l'une des revendications 1, 9 ou 10, dans lequel l'amplitude du mouvement oscillant est inférieure à 500 µm, de préférence inférieure à 300 µm, de préférence encore inférieure à 250 µm.

12. Système de prélèvement de sang selon la revendication 2, dans lequel la lame (13) est déplaçable verticalement au bord tranchant de sorte que ledit bord tranchant (13a) est logé à l'intérieur du boîtier dans une première position et dépasse la surface de contact (16) vers l'extérieur dans une deuxième position.

13. Système de prélèvement de sang selon la revendication 1, 2 ou 12, dans lequel la lame (13) est mue verticalement au bord tranchant par un mécanisme d'avance (25, 26).

14. Système de prélèvement de sang selon l'une des revendications 1 ou 2, dans lequel la lame (13) est fixée sur un élément à ressort (20) tendu et est mue perpendiculairement au bord tranchant à la détente de l'élément à ressort.

15. Système de prélèvement de sang selon l'une des revendications 1 ou 2, dans lequel la lame est bloquée dans le sens de l'incision et dans lequel la surface de contact avec la peau est prévue sur un tube monté élastiquement contre une butée réglable, laquelle permet de régler la profondeur maximale de pénétration de la lame dans le tissu.

16. Système de prélèvement de sang selon la revendication 13, dans lequel le mécanisme d'avance ou l'élément à ressort déplace la lame verticalement au bord tranchant à une vitesse inférieure à 2 km/h, de préférence inférieure à 0,5 km/h, de préférence encore inférieure à 0,2 km/h.

17. Système de prélèvement de sang selon la revendication 2, dans lequel on peut régler la longueur maximale de laquelle la lame dépasse la surface de contact au cours de l'incision et dans lequel la gamme de réglage comprend un intervalle dont la limite inférieure est supérieure à 200 µm et dont la limite supérieure est inférieure à 2500 µm.

18. Système de prélèvement de sang selon la revendication 1, dans lequel la lame possède l'une des formes de base suivantes :
- trapézoïdal
- circulaire ou semi-circulaire
- carré
- polygonal
- pointu ou
- en forme de hachoir.

19. Système de prélèvement de sang selon la revendication 1, dans lequel la lame possède l'un des types d'affûtage suivants:
- affûtage périphérique
- affûtage à facettes
- affûtage et contre-affûtage.

20. Système de prélèvement de sang selon la revendication 1, dans lequel l'oscillateur comporte comme élément oscillant un cristal piézoélectrique (14), une céramique à ultrason ou un transducteur électrodynamique ou un élément oscillant excité mécaniquement et dans lequel cet élément oscillant est commandé par un générateur d'oscillation électronique (15, 17) ou mécanique.

21. Système de prélèvement de sang selon la revendication 1, dans lequel la lame est mise en oscillations par un oscillateur mécanique.

22. Système de prélèvement de sang selon la revendication 1, dans lequel la lame est réalisée en une matière plastique qui est façonnable par un procédé de moulage par injection.

23. Système de prélèvement de sang pour obtenir du sang provenant d'un tissu humain ou animal, comprenant
une lame (33) avec un bord tranchant pointu,
**caractérisé en ce que** la lame est soumise par un oscillateur (32) à un mouvement oscillant essentiellement verticalement par rapport à la surface de tissu, la fréquence du mouvement oscillant étant comprise entre 10 Hz et 10 kHz.

24. Système de prélèvement de sang selon la revendication 23, dans lequel la lame est en forme d'aiguille.

25. Système de prélèvement de sang selon la revendication 23, dans lequel la fréquence du mouvement oscillant est supérieure à 500 Hz, de préférence supérieure à 700 Hz, de préférence encore supérieure à 1000 Hz mais inférieure à 10 kHz.

26. Système de prélèvement de sang selon la revendication 23, dans lequel l'amplitude du mouvement oscillant est inférieure à 300 µm, de préférence inférieure à 100 µm, de préférence encore inférieure à 50 µm.

27. Système de prélèvement de sang selon la revendication 23, possédant un mécanisme de mouvement (25, 26) destiné à mouvoir la lame essentiellement verticalement par rapport à la surface de tissu et commandant le mouvement de la lame de telle manière que le mouvement se transforme en mouvement rétrograde au moment d'atteindre un point mort prédéfini.

28. Système de prélèvement de sang selon la revendication 23, possédant un mécanisme de mouvement (25, 26) pour mouvoir la lame essentiellement verticalement par rapport à la surface de tissu et possédant un guide (10, 23, 41) qui commande le mouvement de la lame pour entrer dans le tissu (aller) et le mouvement pour sortir du tissu (retour) de telle manière que le chemin de la lame est essentiellement le même à l'aller et au retour.

29. Système de prélèvement de sang selon la revendication 23, dans lequel la lame est fixée sur un élément à ressort (20) tendu et dans lequel, à la détente de l'élément à ressort, la lame est mue essentiellement verticalement au bord tranchant.

30. Système de prélèvement de sang selon la revendication 27 ou 29, dans lequel la vitesse verticale de la lame par rapport à la surface de tissu et engendrée par le mécanisme de mouvement ou l'élément à ressort est inférieure à 1 km/h, de préférence inférieure à 0,5 km/h, de préférence encore inférieure à 0,2 km/h.

31. Système de prélèvement de sang selon la revendication 23, dans lequel la lame possède plusieurs bords tranchants pointus.
